# EUROPEAN PATENT SPECIFICATION

(11) **EP 1 055 667 B1**
(45) Date of publication and mention of the grant of the patent: **12.11.2003**
(21) Application number: 00110792.9
(22) Date of filing: 20.05.2000
(51) Int. Cl.: C07C 323/12, A23L 1/226, A23L 1/03

(54) **Mercapto alcohols as flavouring compounds**
Mercaptoalkohol-Verbindungen als als Geschmackstoffe
Mercaptoalcools comme aromatisants

(30) Priority: 28.05.1999 EP 99110416
(43) Date of publication of application: 29.11.2000
(73) Proprietor: Givaudan SA, 1214 Vernier-Genève (CH)
(72) Inventor: Acuna, Gonzalo, 8953 Dietikon (CH); Gautschi, Markus, 4314 Zeiningen (CH); Kumli, Frank, 5015 Niedererlinsbach (CH); Schmid, Joachim, 8604 Volketswil (CH); Zsindely, Janos, 8484 Weisslingen (CH)
(74) Representative: Patentanwälte Schaad, Balass, Menzl & Partner AG

(56) References cited:
- EP-A- 0 924 198
- GB-A- 1 423 914
- US-A- 3 892 878
- US-A- 3 970 689
- OLSEN, ARNE: "onion-like off-flavor in beer : isolation and identification of the culprits" STN CAPLUS, XP002120546
- K-H-ENGEL ET AL: "Identification of new sulfur-containing volatiles in yellow passion fruits (Passilflora edulis f.flavicarpa)" JOURNAL OF AGRICULTURAL AND FOOD CHEMISTRY,US,AMERICAN CHEMICAL SOCIETY. WASHINGTON, vol. 39, no. 12, December 1991 (1991-12), pages 2249-2252, XP002100629 ISSN: 0021-8561

## Description

The present invention relates to new 3-mercapto-alkanols, namely 3-mercapto-2-methyl-butan-1-ol and its diastereoisomers, to flavoring compositions and to food or beverage products flavored with at least one of these compounds and to a method for the preparation of both diastereoisomers of 3-mercapto-2-methyl-butan-1-ol.

In the food and beverage industry flavors play a critical role in the appreciation of food and beverage products. Hereafter the term flavor shall comprise as well flavor, as also aroma and/or taste and in the following context all these terms are used interchangeably.

Several mercapto-alkan-1-ol compounds have been identified as flavorant in food products. Thus, 3-mercapto-hexan-1-ol has been identified in the yellow passion fruit (*Passiflora edulis* f. flavicarpa) (K. -H. Engel, R. Tressel, *J. Agric. Food Chem.* **1991**, *39*, 2249), in Sauvignon blanc wine (T. Tominaga, A. Furrer, R. Henry, D. Dubourdieu, *Flavour Fragrance J.* **1998,** *13,* 159; P. Werkhoff, M. Güntert, G. Krammer, H. Sommer, J. Kaulen, *J. Agric. Food Chem.* **1998,** 46, 1076) and in red Bordeaux wine (P. Bouchilloux, P. Darriet, R. Henry, V. Lavigne-Cruège, D. Dubourdieu, *J. Agric. Food Chem*. **1998**, *46*, 3095) and is usually described as having passion fruit and grapefruit character.

3-mercapto-3-methyl-butan-1-ol has been found in roasted coffee (W. Holscher, O.G. Vitzthum, H. Steinhart, *J. Agric. Food Chem.* **1992,** *40,* 655) and in Sauvignon blanc wine (T. Tominaga, A. Furrer, R. Henry, D. Dubourdieu, *Flavour Fragrance J*. **1998,** *13*, 159). The flavor description by Holscher et al. is sweet, soup-like.

3-mercapto-2-methyl-propan-1-ol has been identified in red Bordeaux wine (P. Bouchilloux, P. Darriet, R. Henry, V. Lavigne-Cruège, D. Dubourdieu, *J. Agric. Food Chem.* **1998,** *46*, 3095) and has been described as broth, sweat-like.

The German patent DE-2316456 describes γ-mercapto-alcohols and their formate and acetate esters as important odorants and flavorants that are useful for the preparation and modification of a broad range of flavor compositions. These compounds have been described as faint green, onion-like, sulfury and sweaty with a broad range of taste thresholds.

An object of the present invention is to provide the two diastereoisomeric forms of 3-mercapto-2-methyl-butan-1-ol, namely (*u*)-3-mercapto-2-methyl-butan-1-ol of formula III 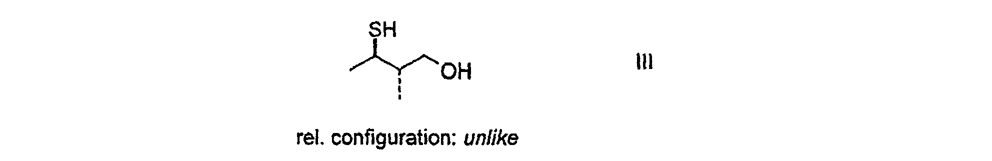 and (*l*)-3-mercapto-2-methyl-butan-1-ol of formula IV.

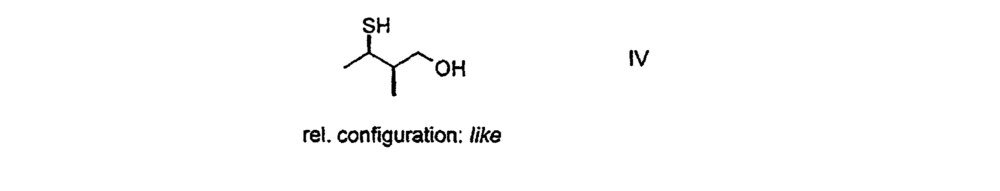

It has been found that the (*u*)-3-mercapto-2-methyl-butan-1-ol enantiomers of formula III exhibit a strong onion-like note, with a surprisingly extreme low odor threshold value of 4 pg/l air. Due to this extremely strong aroma (*u*)-3-mercapto-2-methyl-butan-1-ol of formula III is a valuable flavor ingredient, and even at concentrations of down to 0,01 ppb in water, the flavor of these compounds can be recognized. It has been found that the enantiomers of formula III are useful to enhance the meaty, boiled meat character of a food product, especially meat products, at concentrations of 0.1 ppb to 1 ppb and to impart cooked vegetable and meaty notes to food products, especially soup products, at concentrations from 1ppb to 100 ppb.

It has been further found that the (*l*)-3-mercapto-2-methyl-butan-1-ol enantiomers of formula IV exhibit a herbaceous, onion-like, leeky and gassy character with a far higher threshold value of about 400 pg/l air as compared to the enantiomers of formula III. Surprisingly, it has been found that this compound is useful to enhance the typical natural, fruity character of exotic fruits at concentrations of 10 ppb to 50 ppm in a food product, preferably at concentrations in the range of 100 ppb to 5 ppm.

The compounds of formulae III and IV are not limited to any particular isomer; all possible enantiomers and all mixtures are thus included within the scope of the invention.

The 3-mercapto-alkanols of the present invention are useful for flavoring various products such as foods, beverages, chewing gums, oral hygiene products, and pharmaceuticals, but are especially favored for flavoring foods and beverages. The 3-mercapto-alkan-1-ols according to the present invention can be added directly to the product or preferably as flavor composition comprising usual additives that are well known to a person of skill in the art. The 3-mercapto-alkan-1-ols according to the present invention can also be used in flavoring compositions in order to enhance or modify existing flavors in order to provide a specific flavor impression. They may then be incorporated into the flavoring compositions exclusively or in combination with further flavor ingredients such as esters, aldehydes, ketones, alcohols, lactones, heterocycles as e.g. furans, pyridines, pyrazines, and other sulfur compounds as e.g. thiols, sulfides, disulfides and the like. These components can be combined in proportions normally used in the art for the preparation of flavoring and a person of skill in the art is well aware how to make use of these.

It may be desirable preparing the flavoring compositions according to the present invention by using carrier materials, e.g. gum arabic or maltodextrin, or solvents, e.g. ethanol, propyleneglycol, water or triacetin, yielding inter alia emulsions. By using carrier materials or solvents the desired physical form of the flavoring composition can be obtained. When the carrier materials build an emulsion, the flavoring composition may further contain emulsifiers such as mono- and diglycerides of fatty acids and the like. The flavoring composition according to the invention may be used in spray-dried, liquid, encapsulated, emulsified or other form.

The 3-mercapto-alkan-1-ols of the present invention may be used solely or in combination with other flavor ingredients known by those skilled in the art. Thus, a flavor composition may contain one or more of the compounds according to the invention. The total content of one or more of these compounds is preferably in the range of 0,01 ppb to 50 ppm, preferably in the range of 1 ppb to 5 ppm, depending on the product which should be flavored.

A further object of the present invention is to provide a procedure for the stereospecific synthesis of the 2 diastereoisomeric forms of 3-mercapto-2-methyl-butan-1-ol of formula I, namely (*u*)-3-mercapto-2-methyl-butan-1-ol of formula III and (*l*)-3-mercapto-2-methyl-butan-1-ol of formula IV. The procedure for the preparation of (*u*)-3-mercapto-2-methyl-butan-1-ol comprises the condensation of benzyl mercaptan with angelic acid methyl ester, the reduction of the formed ester with lithium aluminum hydride and subsequent debenzylation with sodium in liquid ammonia. Using this procedure (*u*)-3-mercapto-2-methyl-butan-1-ol is obtained with a diastereoisomeric purity of >6:1. The isomerically enriched product can be used as such as flavorant to enhance the flavor properties of a food product, or it can be purified by chromatography to get diastereomerically pure (*u*)-3-mercapto-2-methyl-butan-1-ol, to be used as flavorant as described infra.

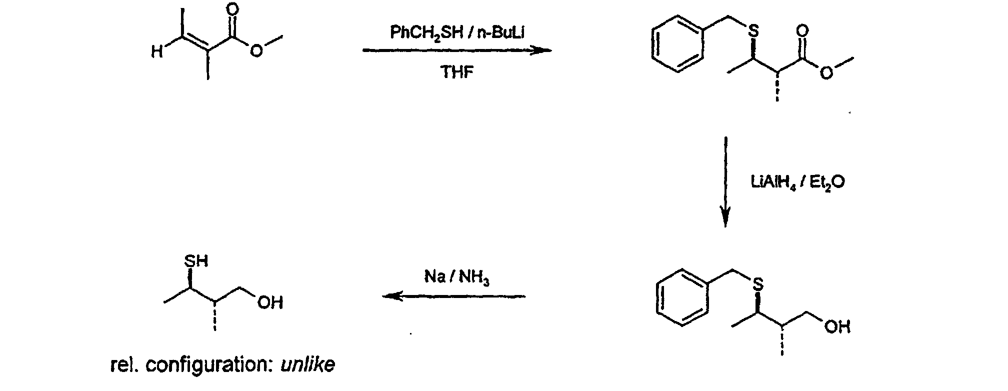

The procedure for the preparation of (*u*)-3-mercapto-2-methyl-butan-1-ol comprises the condensation of benzyl mercaptan with tiglic acid methyl ester, the reduction of the formed ester with lithium aluminum hydride and subsequent debenzylation with sodium in liquid ammonia. Using this procedure (*l*)-3-mercapto-2-methyl-butan-1-ol is obtained with a diastereoisomeric purity of >30:1. The isomerically enriched product can be used as such as flavorant to enhance the flavor properties of a food product, or it can be purified by chromatography to get diastereomerically pure (*l*)-3-mercapto-2-methyl-butan-1-ol, to be used as flavorant as described infra.

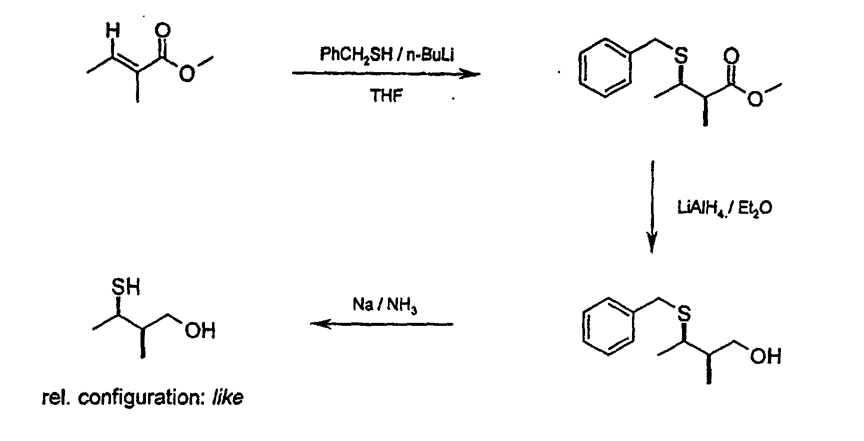

The present invention is described further in the following examples showing specific embodiments, which are presented solely for the non-limiting purpose of further illustrating the invention.

### Example 1

### Preparation of (l)-3-mercapto-2-methyl-butan-1-ol (racemic)

### (a) (l)-3-benzylsulfanyl-2-methyl-butyric acid methyl ester

At a temperature of 0° C to a solution of 13.0 ml of *n*-BuLi (1.6 M in hexane) in 500 ml of THF was added under stirring 236 ml (2.0 mol) of benzyl mercaptan. Then a solution of 23.6 g (0.2 mol) of tiglic acid methyl ester in 500 ml of THF was added. The reaction mixture was allowed to warm up to room temperature and stirring was continued for 3.5 h. The reaction mixture was quenched with 200 ml of a 5% NaOH solution and the organic layer was separated and dried over MgSO₄. THF and benzyl mercaptan were distilled off *in vacuo* (rotary evaporator, 10 mbar/60° C) and the residue was distilled (0.1 mbar, 110-118° C) and gave 40.8 g (85.7%) (*l*)-3-benzylsulfanyl-2-methyl-butyric acid methyl ester in form of a colorless oil having a diastereoisomeric purity of 98:2 (NMR).
- NMR (CDCl₃):: 1.22 (d, CH₃), 1.28 (d, CH₃), 2.57 (m, CH), 2.96 (m, CH), 3.64 (s, CH₃), 3.72 (s, CH₂), 7.15-7.35 (m, 5 arom. H) ppm.
- MS:: 238 (3, M⁺), 151 (3), 147 (15), 123 (27), 91(100), 59 (18).
- IR (neat):: 3028w, 2977m, 2950m, 1736s, 1495m, 1453s, 1200m.

### (b)(l)-3-benzylsulfanyl-2-methyl-butan-1-ol

At a temperature of 0° C to a suspension of 4.78 g (126 mmol) of LiAlH₄ in 150 ml of Et₂O was added a solution of 20.0 g (84 mmol) of (*l*)-3-benzylsulfanyl-2-methyl-butyric acid methyl ester of step (a) in 100 ml of Et₂O. The reaction mixture was allowed to warm up to room temperature, stirring was continued for 5 h. Then H₂O was slowly added until no more H₂ evolution was observed. The reaction mixture was filtrated over Celite, the organic layer was dried over MgSO₄ and concentrated *in vacuo*. Distillation of the crude product at reduced pressure (0.05 Torr/110° C) gave 15.5 g (87%) of (*l*)-3-benzylsulfanyl-2-methyl-butan-1-ol.
- NMR (CDCl₃):: 0.89 (d, CH₃), 1.29 (d, CH₃), 1.60 (t, OH), 1.87 (m, CH), 2.84 (qd, CH), 3.37-3.68 (m, CH₂), 3.74 (d, CH₂), 7.18-7.38 (m, 5 arom. H) ppm.
- MS:: 210 (6, M⁺), 151 (7), 123 (9), 91 (100), 45 (17), 31 (8).
- IR (neat):: 3381br, 3028w, 2961s, 2921s, 2875s, 1494m, 1452s, 1029s.

### (c) (l)-3-mercapto-2-methyl-butan-1-ol

About 200 ml of NH₃ were condensed from a cylinder into a cold flask at -78° C and small pieces of Na were added until a blue color persisted. Then under stirring 3.47 g (64 mmol) of (*l*)-3-benzylsulfanyl-2-methyl-butan-1-ol were added in small portions. Since the reaction mixture became colorless, the Na addition was continued until a blue color persisted again. Stirring at -78° C was continued for 1 h, then the mixture was quenched with saturated NH₄Cl solution until it became colorless, acidified with 100 ml 2N HCl and extracted with Et₂O. The combined organic layers were dried over MgSO₄ and concentrated *in vacuo*. The crude product was distilled at reduced pressure over a 10 cm *Vigreux*-column (0.15 Torr/60° C) and gave 5.8 g (86%) (*l*)-3-mercapto-2-methyl-butan-1-ol in form of a colorless oil having a diastereoisomeric purity of 40:1 (NMR).
- NMR (CDCl₃):: 0.90 (d, CH₃), 1.28 (d, SH), 1.37 (d, CH₃), 1.72 (s, OH), 1.86 (m, CH), 3.28 (m, CH), 3.60 (m, CH₂) ppm.
- MS:: 120 (12, M⁺), 102 (3), 86 (55), 71 (60), 61 (89), 45 (89), 41 (100), 31 (83).
- IR (neat):: 3356br, 2964s, 2926s, 2876s, 1450m, 1379m, 1039s.
- main flavor:: herbaceous, onion-like, leeky, gassy

### Example 2

### Preparation of (u)-3-mercapto-2-methyl-butan-1-ol (racemic)

### (a) (u)-3-benzylsulfanyl-2-methyl-butyric acid methyl ester

At a temperature of 0° C to a solution of 13.0 ml of *n*-BuLi (1.6 M in hexane) in 500 ml of THE was added 236 ml (2.0 mol) of benzyl mercaptan. Then a solution of 23.6 g (0.2 mol) of angelic acid methyl ester in 500 ml of THF was added. The reaction mixture was stirred at 0° C for 3.5 h, quenched with 200 ml of a 5% NaOH solution. The organic layer was separated and dried over MgSO₄. THF and benzyl mercaptan were distilled off *in vacuo* (rotary evaporator, 10 mbar/60° C) and the residue was distilled (0.1 mbar, 104-130° C) and gave 28.2 g (59%) (*u*)-3-benzylsulfanyl-2-methyl-butyric acid methyl ester in form of a colorless oil having a diastereoisomeric purity of 9:1 (NMR).
- NMR(CDCl₃):: 1.18 (d, CH₃), 1.20 (d, CH₃), 2.65 (m, CH), 3.03 (m, CH), 3.66 (s, CH₃), 3.72 (s, CH₂), 7.15-7.35 (m, 5 arom. H) ppm.
- MS:: 238 (3, M⁺), 151 (2), 147 (12), 123 (22), 91 (100).
- IR (neat):: 3029w, 2975m, 1736s, 1495m, 1453s, 1199m.

### (b) (u)-3-benzylsulfanyl-2-methyl-butan-1-ol

At a temperature of 0° C to a suspension of 4.78 g (126 mmol) of LiAlH₄ in 150 ml of Et₂O was added under stirring a solution of 20.0 g (84 mmol) of (*u*)-3-benzylsulfanyl-2-methyl-butyric acid methyl ester in 100 ml of Et₂O. The reaction mixture was allowed to warm up to room temperature and stirring was continued for 5 h. Then H₂O was slowly added until no more H₂ evolution was observed. The reaction mixture was filtrated over Celite, the organic layer was dried over MgSO₄ and concentrated *in vacuo*. Distillation of the crude product at reduced pressure (0.05 Torr/100° C) gave 14.7 g (83%) of (*u*)-3-benzylsulfanyl-2-methyl-butan-1-ol having a diastereoisomeric purity of 6.5:1 (NMR).
- NMR (CDCl₃):: 0.95 (d, CH₃), 1.22 (d, CH₃), 1.73 (t, OH), 1.86 (m, CH), 2.78 (m, CH), 3.53 (m, CH₂), 3.74 (d, CH₂), 7.18-7.38 (m, 5 arom. H) ppm.
- MS:: 210 (6, M⁺), 151 (7), 123 (9), 91 (100), 45 (17).
- IR (neat):: 3377br, 3028w, 2963s, 2923s, 2875s, 1494m, 1452s, 1029s.

### (c) (u)-3-benzylsulfanyl-2-methyl-butan-1-ol

About 200 ml of NH₃ were condensed into a flask at -78° C and small pieces of Na were added until a blue color persisted. Then under stirring 9.5 g (40 mmol) of (*u*)-3-benzylsulfanyl-2-methyl-butan-1-ol of step (b) were added in small portions. Since the reaction mixture became colorless, Na addition was continued until a blue color persisted again. Stirring at -78° C was continued for 1 h, the mixture was quenched with saturated NH₄Cl solution until it became colorless, acidified with 100 ml 2N HCl and extracted with Et₂O. The combined organic layers were dried over MgSO₄ and concentrated *in vacuo*. The crude product was distilled at reduced pressure over a 10 cm *Vigreux*-column (0.06 Torr/47° C) and gave 3.0 g (62%) (*u*)-3-mercapto-2-methyl-butan-1-ol in form of a colorless oil having a diastereoisomeric purity of 6.5:1.
- NMR (CDCl₃):: 1.00 (d, CH₃), 1.35 (d, CH₃), 1.51 (d, SH), 1.78 (m, CH), 1.97 (br, OH), 3.08 (m, CH), 3.65 (d, CH₂).
- MS:: 120 (13, M⁺), 102 (4), 86 (58), 71 (62), 61 (90), 45 (79), 41 (100), 31 (76).
- IR (neat):: 3353br, 2965s, 2928s, 2876s, 1449m, 1379m, 1034s.
- main flavor:: strong onion-like, brothy character

### Example 3

### Flavor tests in food

A meat base having the following composition (parts by weight), was prepared:

| | |
|---|---|
| water | 10 |
| HVP | 30 |
| thiamin HCl | 10 |
| smoke extract | 50 |
| total | 100 |

With this meat base the following bouillons were prepared:

| **Bouillon A** (reference) | |
|---|---|
| starting material: bouillon fat free | |
| added thereto: | |
| meat base | 100 ppm |

| **Bouillon B** | |
|---|---|
| starting material: bouillon fat free | |
| added thereto: | |
| meat base | 100 ppm |
| (*rac*)-(*u*)-3-mercapto-2-methyl-butan-1-ol | 1 ppb |

The bouillons A and B have been compared in a blind test by an expert panel of 6 flavorists. The panel judged the bouillon B to have a fuller, more bouillon-like aroma with increased meaty and fatty notes.

Thus, in this test the present flavor components enhance the bouillon or meat character.

## Claims

1. 3-mercapto-2-methyl-butan-1-ol having the formula 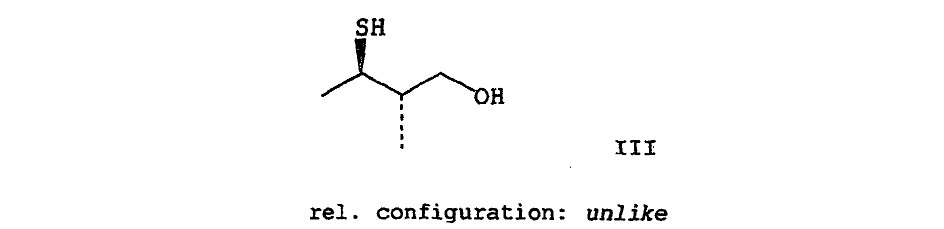 and the enantiomer thereof.

2. 3-mercapto-2-methyl-butan-1-ol having the formula 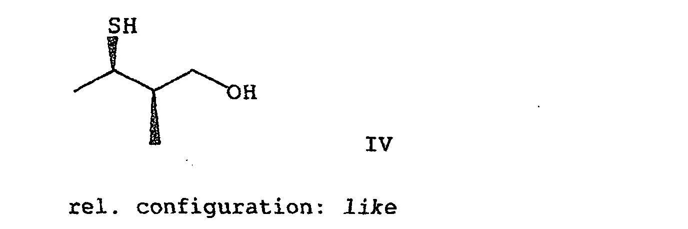 and the enantiomer thereof.

3. 3-mercapto-2-methyl-butan-1-ol enriched in a ratio of more than 6:1 in the diastereomer having the formula

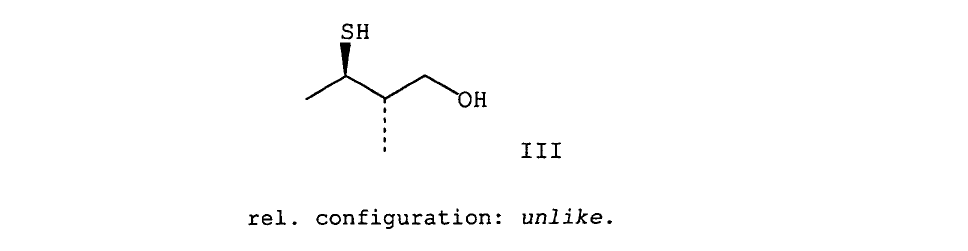

4. 3-mercapto-2-methyl-butan-1-ol enriched in a ratio of more than 30:1 in the diastereomer having the formula

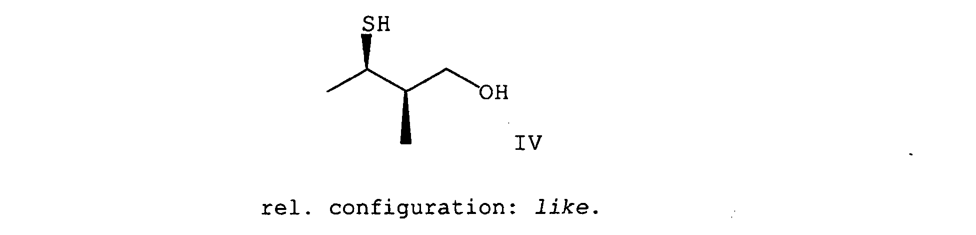

5. Use of a compound of any one of the claims 1 to 4 as flavorant.

6. A flavor composition containing at least one of the compounds of claims 1 to 4.

7. The flavor composition of claim 6 containing a stereoisomer of the compound of claim 1 or mixtures thereof.

8. The flavor composition of claim 6, containing a stereoisomer of the compound of claim 3 or mixtures thereof.

9. A flavor composition of any one of the claims 6 to 8 containing altogether one or more of the compounds in the range of about 0,01 ppb to 50 ppm.

10. A food or beverage product containing a flavor composition of any one of the claims 6 to 8.

## Patentansprüche

1. 3-Mercapto-2-methyl-butan-1-ol der Formel 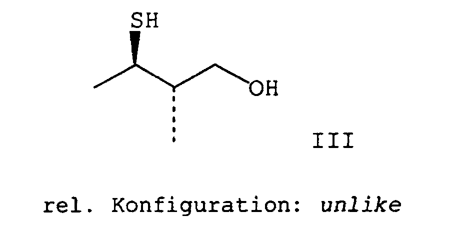 und das Enantiomere davon.

2. 3-Mercapto-2-methyl-butan-1-ol der Formel 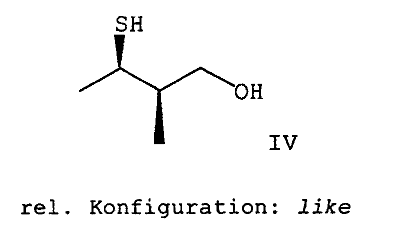 und das Enantiomere davon.

3. 3-Mercapto-2-methyl-butan-1-ol angereichert mit dem Diastereomer der Formel 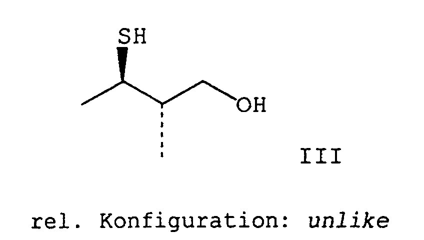 in einem Verhältnis von mehr als 6:1.

4. 3-Mercapto-2-methyl-butan-1-of angereichert mit dem Diastereomer der Formel 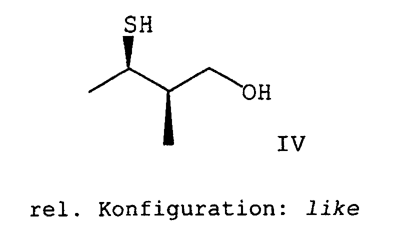 in einem Verhältnis von mehr als 30:1.

5. Verwendung einer Verbindung irgendeines der Ansprüche 1 bis 4 als Aromastoff.

6. Eine Aromazusammensetzung beinhaltend wenigstens eine der Verbindungen nach den Ansprüchen 1 bis 4.

7. Die Aromazusammensetzung nach Anspruch 6 beinhaltend ein Stereoisomer der Verbindung nach Anspruch 1 oder Mischungen davon.

8. Die Aromazusammensetzung nach Anspruch 6 beinhaltend ein Stereoisomer der Verbindung nach Anspruch 3 oder Mischungen davon.

9. Eine Aromazusammensetzung nach irgendeinem der Ansprüche 6 bis 8, welche insgesamt eine oder mehrere der Verbindungen im Bereich von zwischen ca. 0.01 ppb bis 50 ppm beinhaltet.

10. Ein Nahrungsmittel- oder Getränkeprodukt beinhaltend eine Aromazusammensetzung nach irgendeinem der Ansprüche 6 bis 8.

## Revendications

1. 3-Mercapto-2-méthyl-butan-1-ol de formule 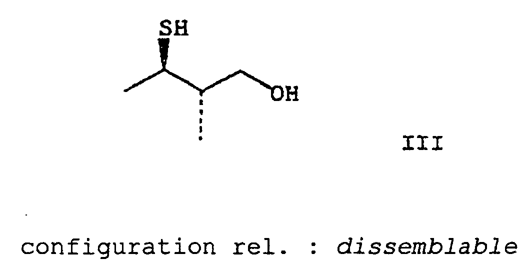 et son énantiomère.

2. 3-Mercapto-2-méthyl-butan-1-ol de formule 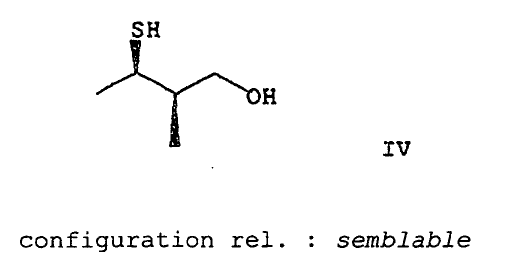 et son énantiomère.

3. 3-Mercapto-2-méthyl-butan-1-ol enrichi en proportion de plus de 6:1 en le diastéréo-isomère de formule

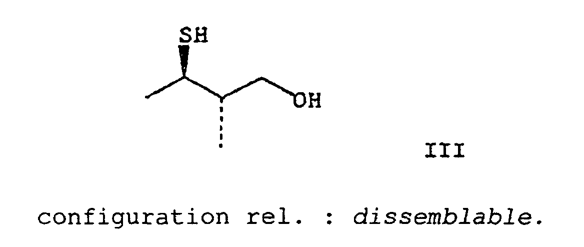

4. 3-Mercapto-2-méthyl-butan-1-ol enrichi en proportion de plus de 30:1 en le diastéréo-isomère de formule

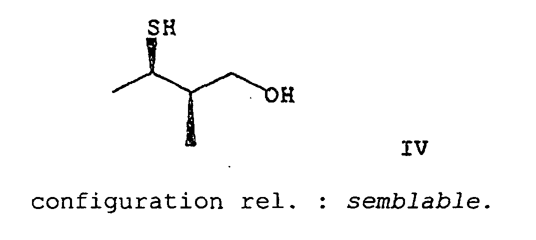

5. Utilisation d'un composé selon l'une quelconque des revendications 1 à 4, comme arôme.

6. Composition aromatique contenant au moins l'un des composés selon les revendications 1 à 4.

7. Composition aromatique selon la revendication 6, contenant un stéréo-isomère du composé selon la revendication 1 ou ses mélanges.

8. Composition aromatique selon la revendication 6, contenant un stéréo-isomère du composé selon la revendication 3 ou ses mélanges.

9. Composition aromatique selon l'une quelconque des revendications 6 à 8, contenant au total un ou plusieurs des composés dans la gamme d'environ 0,01 ppb à 50 ppm.

10. Produit alimentaire ou de boisson contenant une composition aromatique selon l'une quelconque des revendications 6 à 8.
